# EUROPÄISCHE PATENTANMELDUNG

(11) **EP 2 606 920 A1**
(43) Veröffentlichungstag der Anmeldung: **26.06.2013**
(21) Anmeldenummer: 11075273.0
(22) Anmeldetag: 22.12.2011
(51) Int. Cl.: A61M 1/10, A61M 1/12, A61M 25/06

(54) **Schleuseneinrichtung zum Einführen eines Katheters**

(71) Anmelder: ECP Entwicklungsgesellschaft mbH, 12247 Berlin (DE)
(72) Erfinder: Töllner, Thomas, 12047 Berlin (DE); Decke, Robert, 12247 Berlin (DE)
(74) Vertreter: Pfenning, Meinig & Partner GbR

(57) **Zusammenfassung**

Gemäß der Erfindung ist eine Schleuseneinrichtung zum Einführen eines Katheters in einen Patientenkörper mit einer ersten Schleuse (10, 11, 13, 21, 21', 21", 41, 43, 43') mit einem proximalen Ende und einem distalen Ende vorgesehen, wobei beim bestimmungsgemäßen Gebrauch das distale Ende der ersten Schleuse zur Anordnung im Patientenkörper und das proximale Ende der ersten Schleuse zur Anordnung außerhalb des Patientenkörpers vorgesehen ist und wobei die erste Schleuse einen schlauchförmigen Abschnitt (11, 21, 21', 21" , 41) und ein an dessen proximalem Ende angeordnetes Schleusengehäuse (13, 43) mit einem Aufnahmekanal (46) für einen strangförmigen Körper (20', 20" , 20''', 32, 66) aufweist.

Die Erfindung löst das Problem eines zuverlässigen Fixierens einer zweiten Schleuse bzw. eines Katheters gegenüber der ersten Schleuse dadurch, dass an dem Aufnahmekanal (46) eine Klemmvorrichtung (54, 54', 55, 56, 56', 57, 57') zum Fixieren eines strangförmigen Körpers in dem Aufnahmekanal durch Klemmen vorgesehen ist.

## Beschreibung

Die Erfindung liegt auf dem Gebiet der Mechanik und speziell der Medizintechnik und bezieht sich auf eine Schleuseneinrichtung zum Einführen eines Katheters in den Körper eines Patienten.

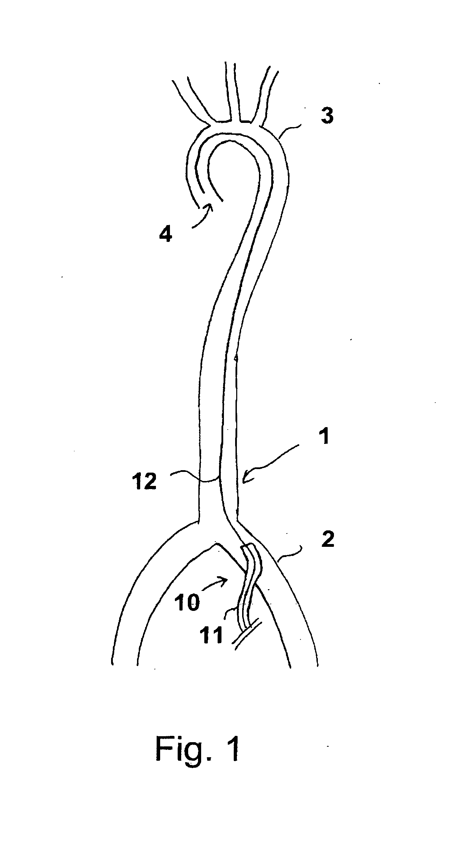

Insbesondere in der mikroinvasiven oder minimalinvasiven Anwendung im Bereich der Medizin werden häufig Funktionselemente wie beispielsweise Stents, Fräsköpfe zum Ausfräsen von Blutgefäßen sowie Herzunterstützungspumpen mittels Kathetern durch eine Öffnung in das Körperinnere eines Patienten, speziell in körpereigene Gefäße, insbesondere Blutgefäße, eingeführt. Um solche Katheter mit minimierter Traumatisierung des betroffenen Gewebes und auch unter Minimierung des Beschädigungsrisikos für die empfindlichen medizinischen Geräte ein- oder mehrmals einführen zu können, werden häufig Schleusen verwendet, die dauerhaft oder vorübergehend in den Körper eines Patienten eingebracht werden und die ein inneres Lumen aufweisen, das das Durchführen eines Katheters oder anderer Funktionselemente erlaubt. Zum Einführen einer solchen Schleuse in den Patientenkörper ist die sogenannte Seldinger-Technik bekannt, die weiter unten im Zusammenhang mit der Figur 1 näher erläutert wird.

Ein entsprechendes Verfahren ist beispielsweise aus der WO 02/43791 A1 bekannt.

Ist eine derartige Schleuse einmal installiert, so kann sie dauerhaft oder wiederholt zum Einführen und Entnehmen eines Katheters verwendet werden.

Eine spezielle Anwendung liegt in der Verwendung einer derartigen Schleuse für komprimierbare Blutpumpen oder andere Funktionselemente, die zunächst zur besseren Einbringbarkeit in den Körper komprimiert, insbesondere radial komprimiert werden, im komprimierten Zustand durch die Schleuse an den Ort des Einsatzes oder bis in die Nähe des Einsatzortes eingeführt und danach dort expandiert werden. Zu diesem Zweck sind entsprechende Blutpumpen bekannt, die einen Pumpenkopf am distalen Ende eines Hohlkatheters aufweisen, wobei der Pumpenkopf einen Rotor mit radial expandierbaren Förderschaufeln sowie ein ebenfalls komprimier- und expandierbares Gehäuse aufweist. Die entsprechenden Elemente sind so ausgebildet, dass sie beispielsweise selbsttätig expandieren, wenn sie beispielsweise vorher elastisch komprimiert sind, oder dass sie zu Beginn eines Rotationsbetriebs durch den Widerstand der zu fördernden Flüssigkeit, beispielsweise des Blutes, expandiert werden (gilt insbesondere für Förderschaufeln des Rotors).

Auch andere Effekte wie beispielsweise Formgedächtniseffekte bei sogenannten Formgedächtnislegierungen, beispielsweise Nitinol, können für den Zweck einer nachträglichen Formänderung ausgenutzt werden.

Entsprechende komprimierbare Blutpumpen sind beispielsweise aus der WO 02/43791 A1 oder aus der EP 2 047 872 A1 sowie auch aus der DE 100 59 714 bekannt.

Solche Blutpumpen oder auch andere Funktionselemente können zur besseren Einbringbarkeit mittels einer oben beschriebenen Schleuse vorteilhaft vorkomprimiert werden und beispielsweise in einer zweiten Schleuse für die Behandlung eines Patienten vor dem Einführen in die erste Schleuse bereitgehalten werden. Die zweite Schleuse ist dann derart konzipiert, dass das Funktionselement, insbesondere der Pumpenkopf, in ihrem Innenraum (Lumen) komprimiert gehalten ist, beispielsweise in demselben Durchmesser, der auch für das Einführen in die erste Schleuse benötigt wird, oder geringfügig größer. Zum Überführen der Pumpe von der zweiten Schleuse in die erste Schleuse werden dann typischerweise die beiden Schleusen derart gekoppelt, dass sie koaxial zueinander mit möglichst geringem Abstand voneinander gekoppelt werden, um die Pumpe aus der zweiten Schleuse heraus und in die erste Schleuse hinein axial zu verschieben.

Dieser Vorgang ist kritisch, da alle Bauteile, insbesondere der Katheter, der Pumpenkopf und die zweite Schleuse mechanisch besonders beansprucht werden. Zudem besteht ein Problem darin, dass dieser Schleusenvorgang unter Operationsbedingungen üblicherweise von Hand mit geringen weiteren Hilfsmitteln durchgeführt werden muss, wobei hohe Anforderungen an die Einfachheit des Vorgangs und an die Zuverlässigkeit gestellt werden.

Der vorliegenden Erfindung liegt vor diesem Hintergrund die Aufgabe zugrunde, eine Schleuseneinrichtung der eingangs genannten Art zu schaffen, die mit hoher Zuverlässigkeit, geringer Anfälligkeit, geringem konstruktivem Aufwand und einem geringen Schädigungsrisiko für Patienten und die medizinischen Geräte das Einführen eines Funktionselementes in den Patientenkörper ermöglicht.

Die Aufgabe wird mit den Merkmalen der Erfindung gemäß den Patentansprüchen 1 und 10 und gemäß einem Verfahren nach Patentanspruch 12 gelöst.

Gemäß der Erfindung wird eine Schleuseneinrichtung zum Einführen eines Katheters in einen Patientenkörper mit einer ersten Schleuse geschaffen, deren proximales Ende beim bestimmungsgemäßen Gebrauch zur Anordnung außerhalb des Patientenkörpers vorgesehen ist, während das distale Ende der ersten Schleuse im Gebrauch innerhalb des Patientenkörpers liegt. Die erste Schleuse weist einen schlauchförmigen Abschnitt und an dessen proximalem Ende ein Schleusengehäuse mit einem Aufnahmekanal für einen strangförmigen Körper, insbesondere einen Katheter und/oder eine zweite Schleuse, auf.

Durch Vorsehen einer Klemmvorrichtung an dem Aufnahmekanal kann der strangförmige Körper, insbesondere Katheter und/oder eine Schleuse, in dem Aufnahmekanal eingeklemmt werden. Hierdurch kann, wenn ein Funktionselement, insbesondere eine Pumpe, am Ende eines Katheters in einer zweiten Schleuse vorkomprimiert ist, diese zweite Schleuse in das Schleusengehäuse eingeführt und dort mittels der Klemmvorrichtung fixiert werden. Damit können gleichzeitig die zweite Schleuse und die erste Schleuse koaxial zueinander ausgerichtet und in einem gewünschten relativen Abstand axial zueinander fixiert werden, so dass der Katheter mit dem Funktionselement von der zweiten Schleuse in die erste Schleuse hinübergeschoben werden kann.

Die Wandstärke und das Material der zweiten Schleuse werden so ausgewählt, dass die zweite Schleuse in der Klemmvorrichtung eingeklemmt werden kann, ohne dass gleichzeitig der Katheter in der zweiten Schleuse mit eingeklemmt wird. Die zweite Schleuse wird derart stabil ausgeführt, dass sie beim Klemmen nur unwesentlich komprimiert wird. Die Wanddicke der hohlzylindrischen zweiten Schleuse kann vorteilhaft zwischen 0,2 und 1,0 mm, insbesondere zwischen 0,3 und 0,7 mm betragen.

Gleichzeitig wird die Möglichkeit geschaffen, nach dem Einführen des Katheters und/oder des Funktionselements in die erste Schleuse die zweite Schleuse nach dem Lösen der Klemmvorrichtung zu entfernen und diese beispielsweise als Peel-away-Schleuse auszugestalten, so dass sie durch Aufreißen entfernt werden kann, wobei der Katheter in dem Aufnahmekanal bleibt. Der Katheter kann dann trotz seines geringeren Durchmessers in der Klemmvorrichtung geklemmt werden, um das Funktionselement im Patientenkörper relativ zu der ersten Schleuse zu fixieren. Nach einer gewissen Zeit nach dem Einführen, wenn die mechanischen Teile sich gesetzt und die Körpertemperatur des Patienten angenommen haben, kann eine Nachjustierung erforderlich sein, so dass dann die Klemmvorrichtung erneut gelöst, der Katheter neu justiert und die Klemmvorrichtung wieder fixiert werden kann. Alternativ kann auch vorgesehen werden, den Katheter in einer weiteren auf den geringeren Durchmesser abgestimmten Klemmvorrichtung zu fixieren.

Eine vorteilhafte Ausgestaltung der Erfindung sieht vor, dass die Klemmvorrichtung einen elastisch radial deformierbaren Klemmring aufweist. Ein solcher radial deformierbarer Klemmring stellt eine konstruktiv sehr einfache und kostengünstige Art dar, eine Klemmvorrichtung zu schaffen, die ohne die Gefahr einer Verletzung einer zweiten Schleuse oder eines Katheters eine zuverlässige Klemmung gestattet. Der Klemmring kann vorteilhaft aus einem Elastomer bestehen und volumeninkompressibel sein, so dass er bei axialer Kompression radial ausgedehnt wird. Er kann jedoch auch aus einem Schaumstoff bestehen, der teilweise volumenkompressibel ist, sich jedoch ebenso bei einer Axialkompression radial ausdehnt.

Zudem kann vorgesehen sein, dass der Klemmring aus einem Kunststoff oder Metall besteht und geschlitzt ist. Ein derartiger Klemmring kann beispielsweise auch außen eine konische Form aufweisen, um mittels eines weiteren als Klemmkonus ausgebildeten Rings durch dessen axiale Bewegung radial komprimiert werden zu können.

Vorteilhaft kann die Erfindung dadurch ausgestaltet werden, dass der Klemmring mittels einer axial in Bezug auf den Aufnahmekanal wirkenden Schraubvorrichtung in Richtung radial nach innen expandierbar ist. Mittels einer solchen Schraubvorrichtung, beispielsweise mittels eines Schraubrades, dessen Durchmesser auch größer als der des Schleusengehäuses sein kann, kann auch unter Operationsbedingungen in einfacher Weise eine Kompression eines Klemmrings und damit ein Verklemmen der zweiten Schleuse oder des Katheters vorgenommen werden.

Bei Verwendung eines im Querschnitt keilförmigen zylindrischen Druckstücks kann die Erfindung dadurch realisiert werden, dass der Klemmring mittels der axial wirkenden Schraubvorrichtung und eines axial bewegbaren Keilkörpers, insbesondere eines im Querschnitt keilförmigen Rings, radial nach innen komprimiert wird.

Um das Eindringen von Keimen durch die erste Schleuse in den Patientenkörper sowohl bei Einführung einer zweiten Schleuse und eines Katheters als auch später im Betrieb zu minimieren, kann weiter vorgesehen sein, dass auf der in axialer Richtung gesehen dem Druckstück oder dem Keilkörper gegenüberliegenden Seite des Klemmrings eine radiale Erweiterung des Aufnahmekanals angeordnet ist, die eine Spülvorrichtung aufweist.

Zu demselben Zweck und weiterhin, um das Austreten von Körperflüssigkeiten, insbesondere Blut, durch die Schleuse zu verhindern, kann gemäß der Erfindung auch vorgesehen sein, dass in dem Spülraum, insbesondere auf der in axialer Richtung gesehen dem Druckstück oder dem Keilkörper gegenüberliegenden Seite des Klemmrings ein den Aufnahmekanal abdichtendes Ventil angeordnet ist. Ein solches Ventil kann beispielsweise als Doppelventil mit einem Plattenventil und einem Domventil ausgestaltet sein, wobei das Plattenventil dann optimal dichtet, wenn der Aufnahmekanal nicht von einem Katheter und/oder einer zweiten Schleuse durchsetzt ist, so dass das Plattenventil insgesamt geschlossen gehalten werden kann. Wird ein Katheter oder ein anderer strangförmiger Körper durchgeschoben, so erlaubt das Domventil, das beispielsweise die geometrische Form einer Kalotte annehmen kann, eine optimale Dichtung um den strangförmigen Körper herum. Es sind aber auch andere Ausführungen des Ventils möglich um die genannten Abdichtungsfunktionen zu erfüllen.

Die Erfindung bezieht sich außer auf eine Schleuseneinrichtung der oben genannten Art auch auf eine Kathetereinrichtung mit einer entsprechenden Schleuseneinrichtung und mit einem Katheter, der den Aufnahmekanal durchsetzt und in der Klemmvorrichtung fixiert ist.

Weiter soll auch eine Kathetereinrichtung mit einer Schleuseneinrichtung, wie sie oben beschrieben wurde, und einem Katheter sowie einer den Katheter umgebenden zweiten hülsenförmigen Schleuse, die den Aufnahmekanal durchsetzt, Gegenstand der Erfindung sein, wobei die hülsenförmige zweite Schleuse in der Klemmvorrichtung fixiert ist.

Die Erfindung bezieht sich außer auf eine Schleuseneinrichtung und eine Kathetereinrichtung der oben beschriebenen Art auch auf ein Verfahren zum Einführen eines Katheters in einen Patientenkörper mittels einer Schleuseneinrichtung gemäß einer der oben beschriebenen Varianten sowie mit einer zweiten Schleuse, wobei zunächst die zweite Schleuse gemeinsam mit dem Katheter in die erste Schleuse, insbesondere bis zu einem mechanischen Anschlag, eingeführt wird und wobei darauf die zweite Schleuse mittels der Klemmvorrichtung fixiert wird und danach der Katheter von der zweiten Schleuse in die erste Schleuse überführt wird.

Eine vorteilhafte Ausgestaltung des erfindungsgemäßen Verfahrens sieht vor, dass nach dem Einführen des Katheters in die erste Schleuse die zweite Schleuse, insbesondere durch Auf- oder Abreißen, entfernt wird.

Weiter kann das erfindungsgemäße Verfahren vorteilhaft vorsehen, dass nach dem Entfernen der zweiten Schleuse der Katheter in der Klemmvorrichtung unmittelbar fixiert wird.

Die Erfindung wird nachfolgend anhand eines Ausführungsbeispiels in einer Zeichnung gezeigt und anschließend beschrieben. Dabei zeigt
- Fig. 1: eine schematische Übersicht über ein Gefäßsystem mit einer eingeführten ersten Schleuse;
- Fig. 2: eine Detailansicht eines Ausschnitts der Fig. 1;
- Fig. 3: eine Ausführungsform der Erfindung mit einer ersten Schleuse und einer zweiten Schleuse;
- Fig. 4: eine Ausführungsform einer Pumpe;
- Fig. 5: eine zweite Schleuse mit einer aus dieser extrahierten Pumpe;
- Fign. 6,: 7 das Einziehen einer Pumpe in eine zweite Schleuse;
- Fign. 8, 9: das Überführen einer Pumpe von einer zweiten in eine erste Schleuse;
- Fig. 10: einen Längsschnitt durch ein Schleusengehäuse mit einem schlauchförmigen Abschnitt;
- Fig. 11: einen Längsschnitt durch einen Teil eines Schleusengehäuses mit einer Schneidvorrichtung;
- Fig. 12: einen Längsschnitt durch ein Schleusengehäuse mit einer Klemmvorrichtung für den schlauchförmigen Abschnitt und einer weiteren Klemmvorrichtung;
- Fig. 13: einen Längsschnitt durch einen alternativen Klemmring mit einem konischen Druckstück; und
- Fig. 14: einen Längsschnitt durch ein Schleusengehäuse mit einer Klemmvorrichtung für einen proximal eingeschobenen strangförmigen Körper.

In der Fig. 1 ist ein schematisches menschliches Gefäßsystem 1 gezeigt. Im Bereich der Leiste liegt eine der femoralen Arterien 2, die über eine Hauptschlagader mit dem Aortenbogen 3 verbunden ist und anschließend in die Herzkammer 4 mündet. Mithilfe beispielsweise der Seldinger-Technik wird zunächst eine Einführschleuse 10 in die femorale Arterie 2 eingeführt. Hierbei wird zunächst die femorale Arterie bzw. ein beliebiges Blutgefäß beispielsweise mit einer Stahlkanüle mit schneidender Spitze punktiert. Durch die in die Punktur eingeführte Stahlkanüle hindurch wird ein Führungsdraht 12 geschoben und retrograd über den Aortenbogen 3 in den linken Herzventrikel 4 eingeführt. Nach Entfernung der Punktionskanüle wird die als Einführschleuse ausgebildete erste Schleuse 10, welche einen schlauchförmigen Abschnitt 11 sowie gegebenenfalls einen hier nicht dargestellten Dilatator umfasst, auf den Führungsdraht eingefädelt und durch die punktierte Stelle in das Gefäßsystem eingeführt, wobei die Schleuse eine geringe Distanz in das Lumen des Gefäßsystems oder auch bis zum Einsatzort eines einzuführenden Elementes eingeführt wird. Anschließend wird eine Fluidpumpe durch die Einführschleuse 10 in das Gefäßsystem eingeführt.

Der schlauchförmige Abschnitt 11 der ersten Schleuse 10 ist derart in die Arterie eingeführt, dass das proximale Ende der ersten Schleuse 10 außerhalb der femoralen Arterie liegt und somit zum Einführen beispielsweise einer Pumpe genutzt werden kann. So ist es möglich, die Pumpe auf dem Führungsdraht 12 aufzufädeln, um die Pumpe mithilfe des Führungsdrahtes bis in den linken Herzventrikel zu führen.

Es ist auch möglich, den schlauchförmigen Abschnitt 11 der ersten Schleuse 10 durch den Führungsdraht geführt bis in den linken Herzventrikel zu führen und den Führungsdraht 12 anschließend aus der ersten Schleuse zu entfernen. Eine etwaige Pumpeneinheit wird anschließend durch das erste Schleusenlumen bis in die Nähe oder bis in den linken Ventrikel 4 geführt.

Vorliegend wird das Verfahren lediglich anhand des Einführens einer Pumpe in den linken Herzventrikel zum Unterstützen einer Herzfunktion dargestellt. Für den Fachmann ist jedoch leicht erkennbar, dass die Pumpe oder ein anderes Funktionselement auch an andere Stellen im körpereigenen Gefäßsystem angeordnet und eingebracht werden kann.

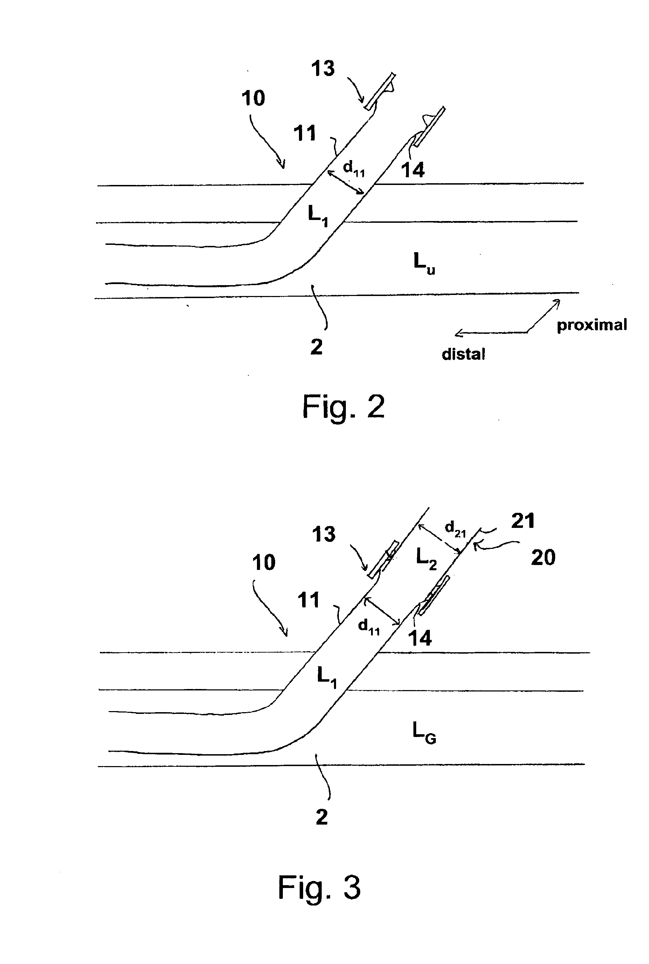

In der Fig. 2 ist der Bereich der Fig. 1 dargestellt, in welchem die erste Schleuse 10 durch das körpereigene Gewebe von außen in das Lumen L_{G} der femoralen Arterie 2 geführt ist. Die erste Schleuse umfasst dabei einen schlauchförmigen Abschnitt 11, welcher proximal mit einem Schleusengehäuse 13 verbunden ist. Der schlauchförmige Abschnitt 11 definiert ein Lumen L₁, welches einen Innendurchmesser d₁₁ aufweist. Zum proximalen Ende des schlauchförmigen Abschnitts 11 hin weitet sich dieser trompetenartig im Bereich 14 auf.

Das Schleusengehäuse 13 enthält ein im Stand der Technik bekanntes hämostatisches Ventil. Dieses verhindert, dass im Lumen L_{G} befindliches Fluid durch das Lumen L₁ nach außen austreten kann.

In der Darstellung der Fig. 3 ist die erste Schleuse 10 der Fig. 2 mit einer zweiten Schleuse 20 gekoppelt. Von der zweiten Schleuse 20 ist lediglich ein schlauchförmiger Abschnitt 21 gezeigt, welcher ein Lumen L₂ mit einem Innendurchmesser d₂₁ definiert. Das distale Ende der zweiten Schleuse 20 weist dabei einen derartigen Außendurchmesser auf, dass es in das Schleusengehäuse 13 eingeführt werden kann. Der Innendurchmesser d₂₁ ist jedoch größer als der Innendurchmesser d₁₁.

Eine nicht dargestellte, im Lumen L₂ befindliche Pumpe kann nun durch Drücken vom zweiten Schleusenlumen L₂ in das erste Schleusenlumen L₁ überführt werden. Anschließend wird die Pumpe durch das erste Schleusenlumen L₁ bis an die Stelle im Gefäßsystem transportiert, an welcher die Pumpe ihre Wirkung entfalten soll. Hierbei kann die Pumpe entweder auf einem Führungsdraht geführt werden oder ohne Führungsdraht durch das erste Schleusenlumen eingebracht werden. Die erste Schleuse kann, um die Pumpe und die Gefäßwände sowie den Wellenkatheter zu schonen, distal bis zum Einsatzort der Pumpe vorgeschoben werden, bevor die Pumpe herausgeschoben wird.

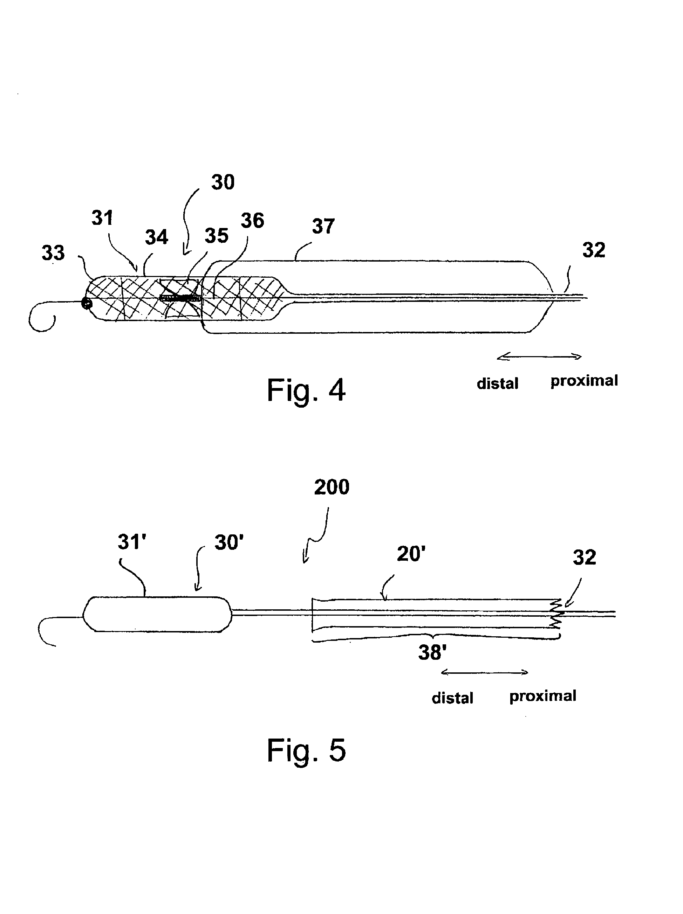

Anhand der Fig. 4 wird eine mögliche Ausführung einer Pumpe 30 genauer erläutert. Die Pumpe 30 umfasst eine distale Pumpeneinheit 31 und einen sich an das proximale Ende der distalen Pumpeneinheit 31 anschließenden Wellenkatheter 32. Der Wellenkatheter 32 weist an seinem proximalen, nicht dargestellten Ende eine Kupplung zur Ankopplung des Wellenkatheters 32 an eine Antriebsvorrichtung auf. Die Antriebsvorrichtung kann außerhalb des Patientenkörpers angeordnet sein und versetzt eine im Wellenkatheter 32 verlaufende flexible Welle in Rotation, welche wiederum die distale Pumpeneinheit 31 antreibt.

Die distale Pumpeneinheit umfasst ein Pumpengehäuse 33, welches aus sich kreuzenden Nitinolstreben hergestellt ist. Das Nitinolgehäuse ist zu Teilen mit einer Beschichtung 34 versehen, welche sich distal und proximal eines im Gehäuse 33 angeordneten Rotors 35 erstreckt. Der Rotor ist mit der durch den Wellenkatheter 32 verlaufenden Welle 36 verbunden und wird so in Drehung versetzt. Das Gehäuse und der Rotor sind komprimierbar, d. h., die Pumpe ist eine selbstentkomprimierbare Pumpe. Die Entfaltung der Pumpe vollzieht sich nach dem Herausschieben der distalen Pumpeneinheit aus dem distalen Ende einer Schleuse. Zum Komprimieren der Pumpe in Vorbereitung der Implantation wird die distale Pumpeneinheit in das distale Ende eines Schleusenlumens einer zweiten Schleuse eingezogen. Das Schleusenlumen besitzt dabei einen Innendurchmesser, welcher zumindest größer als der Außendurchmesser des Wellenkatheters ist.

Der Rotor kann gegenüber dem Pumpengehäuse in Axialrichtung verschiebbar sein, insbesondere mittels einer Axialverschiebung der Antriebswelle. Der Rotor kann andererseits auch in Axialrichtung gegenüber dem Pumpengehäuse fixiert sein.

Optional weist die Pumpe einen Abströmschlauch 37 auf, welcher einen proximal des Rotors 35 gelegenen Strömungskanal für das gepumpte Fluid definiert. Am proximalen Ende des Abströmschlauchs 37 befinden sich nicht näher dargestellte Auslassöffnungen.

Selbstverständlich kann die Pumpe auch von einem Pumpbetrieb in einen Saugbetrieb umgeschaltet werden, so dass die Pumpe nicht länger Fluid vom distalen Ende ans proximale Ende führt, sondern umgekehrt.

Eine detailliertere Beschreibung einer weiteren geeigneten Pumpe kann beispielsweise der Druckschrift EP 2 047 872 A1 entnommen werden.

Anhand der Figuren 5 bis 9 soll nun die Funktion des Systems erläutert werden.

In der Fig. 5 ist eine Pumpe 30' dargestellt, welche im Wesentlichen der Pumpe 30 nach der Fig. 4 entspricht. Zur Vereinfachung sind Details der Pumpe nicht gezeigt. Es sind lediglich das bauchige Gehäuse sowie das distal des bauchigen Gehäuses gelegene "Pigtail" dargestellt, welches ein Ansaugen der Herzpumpe an die Herzwand verhindert. Proximal der distalen Pumpeneinheit 31' verläuft der Wellenkatheter 32'. Einen Bereich 38' des Wellenkatheters 32' umschließend ist eine zweite Schleuse 20' angeordnet, welche ein Lumen L₂ umfasst, dessen Innendurchmesser d₂₁ kleiner als der Durchmesser der distalen Pumpeneinheit 31' im entfalteten Zustand ist.

Die in der Fig. 5 dargestellte Pumpe 30' ist eine komprimierbare Pumpe, das heißt, die distale Pumpeneinheit 31', welche unter anderem das Pumpengehäuse und den darin befindlichen Rotor umfasst, ist derart ausgebildet, dass sie komprimiert, d. h. in ihrem Durchmesser verringert werden kann. Nachdem ein Qualitätsprüfer oder beispielsweise ein Arzt sich von der korrekten Funktion der Pumpe 30' überzeugen konnte, z. B. durch Betrachtung der Rotationsbewegung der in der distalen Pumpeneinheit 31' befindlichen Rotoreinheit bei einem Testlauf, wird durch Ziehen des Wellenkatheters 32' in proximaler Richtung die distale Pumpeneinheit 31' in das Lumen L₂ der zweiten Schleuse 20' gezogen. Durch das Einziehen der Pumpe in die zweite Schleuse 20' wird ein Verbiegen bzw. eine Beschädigung des Wellenkatheters bzw. der darin verlaufenden Welle vermieden. Die in der Fig. 5 dargestellte Pumpe 30' und die den Bereich 38' des Wellenkatheters 32' umschließende zweite Schleuse 20' bilden ein System 200, welches es ermöglicht, rechtzeitig vor einer Operation die Funktion der Pumpe 30' zu testen und anschließend durch das Ziehen der distalen Pumpeneinheit 31' in das distale Ende der zweiten Schleuse 20' die Pumpe zu komprimieren und dabei eine Beschädigung der Welle zu vermeiden. Obgleich das System sowohl mit aktiv entkomprimierbaren Pumpen als auch mit selbstentkomprimierbaren Pumpen realisierbar ist, eignet es sich insbesondere für selbstentkomprimierbare Pumpen, d.h. Pumpen, deren distale Pumpeneinheit außerhalb der Schleuse selbsttätig wieder die ursprüngliche Größe annimmt.

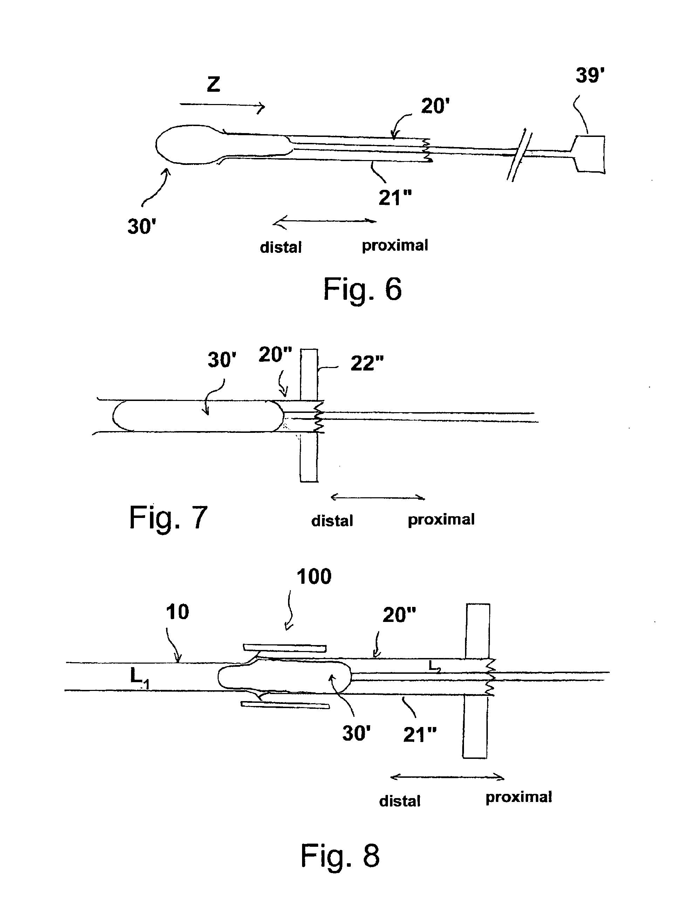

In der Fig. 6 ist ein Zwischenschritt beim Einziehen der distalen Pumpeneinheit 30' in das Lumen der zweiten Schleuse 20' dargestellt. Es ist erkennbar, dass die distale Pumpeneinheit 30' komprimierbar ist und auf einen geringeren Durchmesser gebracht werden kann, so dass die distale Pumpeneinheit 30' in das Lumen der zweiten Schleuse 20' aufgenommen werden kann.

Ferner ist in der Fig. 6 eine an den Wellenkatheter 32' anschließende Kupplung 39' dargestellt, welche ein Ankoppeln der in dem Wellenkatheter verlaufenden Welle an eine Antriebseinheit ermöglicht. Da die Kupplung 39' einen oftmals größeren Außendurchmesser als den Innendurchmesser des Lumens L₂ aufweist, wird die zweite Schleuse 20' zumeist vor der Montage der Kupplung 39' vom proximalen Ende des Wellenkatheters 32' her in distaler Richtung aufgesetzt, so dass die Pumpe im System 200, d. h. die Pumpe mit der sich proximal der distalen Pumpeneinheit 31' befindlichen zweiten Schleuse 20' und der vormontierten Kupplung 39', ausgeliefert wird. An der Fig. 6 ist auch eine leichte Aufweitung des distalen Endes der zweiten Schleuse 20' dargestellt. Die trompetenartige Aufweitung 24' erleichtert das Einziehen der distalen Pumpeneinheit 31' in das Lumen L₂ der zweiten Schleuse 20'.

In der Fig. 7 schließlich befindet sich die distale Pumpeneinheit 31' vollständig im Lumen L₂ der zweiten Schleuse 20 " . Die zweite Schleuse 20 " weist zwei vormontierte Griffeinheiten 22 " auf, welche ein besseres Halten bzw. Entfernen der zweiten Schleuse 20" beim Einziehen der distalen Pumpeneinheit 31' in das Lumen L₂ bzw. ein anschließendes Aufreißen ermöglichen. Vorteilhafterweise wird bei vorhandenem "Pigtail" dieses ebenfalls in das Lumen L₂ eingezogen, so dass sich die distale Pumpeneinheit 31' samt distal der distalen Pumpeneinheit 31' gelegenen Komponenten der Pumpe im Lumen L₂ befindet.

In der Fig. 8 wird erkennbar, wie das System 200 aus Pumpe 30' und zweiter Schleuse 20 " in Wirkverbindung mit der ersten Schleuse 10 zu einem System 100 kombiniert wird. Zunächst wird die zweite Schleuse 20 " mit ihrem distalen Ende in das Schleusengehäuse der ersten Schleuse 10 eingeführt. Sobald die distale Spitze der zweiten Schleuse 20 " an der Mündung des schlauchförmigen Abschnitts der ersten Schleuse 10 anliegt, wird durch Schieben der Pumpe in distaler Richtung, wobei das Schieben anhand eines Schiebens des Wellenkatheters 32' erfolgt, die Pumpe von der zweiten Schleuse 20' in die erste Schleuse 10' überführt. Hierbei wird der Durchmesser der distalen Pumpeneinheit 31' weiter auf den Innendurchmesser d₁₁ des Lumens L₁ reduziert.

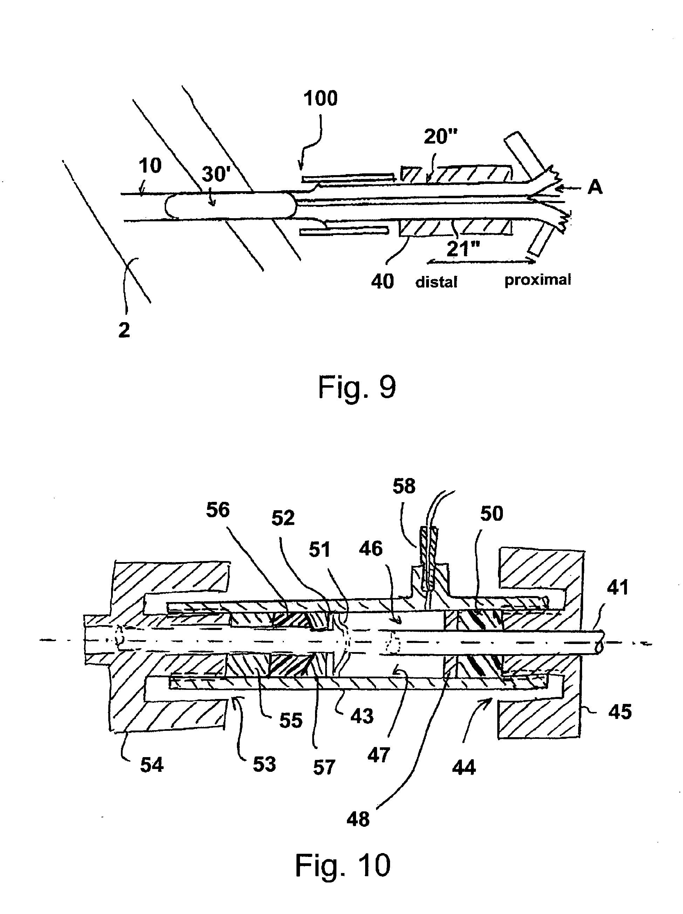

In der Fig. 9 ist der anschließende Schritt dargestellt, bei welchem sich die distale Pumpeneinheit 31' vollständig im Lumen L₁ der ersten Schleuse 10 befindet. Dass sich die distale Pumpeneinheit 31' vollständig im Lumen L₁ der ersten Schleuse 10 befindet, kann beispielsweise anhand einer farblichen Markierung 50, welche auf der Außenseite des Wellenkatheters 32' aufgebracht ist, kenntlich gemacht werden.

Anschließend wird die zweite Schleuse 20", welche als eine "Peel-away"-Schleuse ausgebildet ist, vom Wellenkatheter 32' entfernt, indem die Peel-away-Schleuse vom proximalen zum distalen Ende hin aufgerissen und vom Wellenkatheter 32' abgezogen wird. Das gerichtete Aufreißen vom proximalen zum distalen Ende hin kann durch Einkerbungen A unterstützt werden, beruht jedoch vorwiegend auf der Ausrichtung der Molekülketten des verwendeten Kunststoffs von der proximalen in die distale Richtung.

Nachdem die Peel-away-Schleuse entfernt wurde, wird die Pumpe 30' weiter innerhalb des Lumens L₁ der ersten Schleuse 10 bis zum gewünschten Ort geführt.

Wahlweise kann die erste Schleuse auch vor oder nach dem Einführen der Pumpe mit der distalen Schleusenmündung in die unmittelbare Nähe des Einsatzortes vorgeschoben werden. Hierzu weist die erste Schleuse die notwendige Länge auf.

Eine Versteifung der zweiten Schleuse 20" ist insbesondere beim Einziehen der distalen Pumpeneinheit 31' in das distale Ende des zweiten Schleusenlumens L₂ nicht notwendig, da die Gefahr eines Abknickens der Welle bei einer Ziehbewegung stark reduziert ist.

Beim Überführen der Pumpe von der zweiten Schleuse in die erste Schleuse, wie anhand der Figuren 7 bis 9 dargestellt, kann die zweite Schleuse eine sie verstärkende Struktur in Form eines eingebrachten Drahtes umfassen, oder der schlauchförmige Abschnitt 21" der Schleuse 20" wird nicht aus einem flexiblen Kunststoff, sondern aus einem formfesten Kunststoff oder Metall hergestellt.

Eine weitere Möglichkeit zum Stabilisieren der Pumpe und der zweiten Schleuse besteht darin, beim Vorschieben der Pumpe 30' in distaler Richtung, d.h. insbesondere beim Überführen der Pumpe 30' von der zweiten Schleuse in die erste Schleuse, die zweite Schleuse 20" durch eine Stützvorrichtung 40 in Form einer stabilen äußeren Hülse zu halten.

Anschließend soll noch eine mögliche Variante eines Verfahrens zum Einführen einer Pumpe in einen linken Herzventrikel geschildert werden. Als vorbereitende Maßnahme wird die Pumpe zunächst mit steriler physiologischer Kochsalzlösung befüllt und somit komplett entlüftet. Anschließend wird die proximal der distalen Pumpeneinheit gelegene Peel-away-Schleuse bis zu einem eventuell vorhandenen Abströmschlauch vorgeschoben. Die Peel-away-Schleuse besitzt einen Durchmesser von beispielsweise 10 Fr. Nachdem die Peel-away-Schleuse bis zum Abströmschlauch vorgeschoben wurde, wird die Peel-away-Schleuse von einer hülsenförmigen Vorrichtung zum Halten der zweiten Schleuse umfasst. Anschließend wird die distale Pumpeneinheit, gegebenenfalls unter leichter Drehbewegung, in die Peel-away-Schleuse eingezogen, indem am Wellenkatheter eine Zugbewegung in proximaler Richtung ausgeübt wird. Die Pumpe wird so weit in die zweite Schleuse verschoben, dass ein eventuell vorhandenes Pigtail ebenfalls in der Peel-away-Schleuse geborgen ist. Durch diese Schritte ist es möglich, die Funktionsfähigkeit der Pumpe bereits vor einem operativen Eingriff zu überprüfen und die Pumpe erst anschließend in eine Schleuse einzuführen, ohne unter Zeitdruck agieren zu müssen. Zum Beispiel erst anschließend wird die Punktur des Gefäßsystems zum Einführen der ersten Schleuse durchgeführt. Zur Zeiteinsparung ist es auf diese Weise aber auch möglich, dass ein Assistent die Pumpe vorbereitet während der Anwender bereits parallel die Punktion durchführt.

Nachdem beispielsweise eine 9Fr-Einführschleuse bis in den linken Herzventrikel eingeführt wurde, wird ein eventuell vorhandener Dilatator aus der Einführschleuse gezogen und aus dieser entfernt.

Anschließend wird die in der Peel-away-Schleuse gehaltene Pumpe, welche beispielsweise anfangs von der Hülse zum Halten der zweiten Schleuse umfasst wird, in das Schleusengehäuse eingeschoben, bis die Spitze der Peel-away-Schleuse an einem mechanischen Anschlag anschlägt. Anschließend wird die Pumpe durch Schieben des Wellenkatheters von der Peel-away-Schleuse in den schlauchförmigen Abschnitt überführt. Sobald die distale Pumpeneinheit komplett in die Einführschleuse überführt wurde, wie dies beispielsweise anhand einer optischen Markierung auf dem Wellenkatheterschaft überprüfbar ist, kann die Peel-away-Schleuse aufgerissen und vom Wellenkatheter abgezogen werden. Anschließend wird die Pumpe innerhalb der ersten Schleuse bis in den linken Herzventrikel vorgeschoben. Die erste Schleuse wird anschließend aus dem linken Herzventrikel bis zum Beginn der absteigenden Aorta zurückgezogen.

Die Positionierung der distalen Pumpeneinheit im linken Herzventrikel kann beispielsweise durch Röntgendurchleuchtung kontrolliert werden. Hierzu befindet sich eine röntgensichtbare Markierung am Pumpengehäuse bzw. in dessen Nähe, beispielsweise am Katheter, oder das Pumpengehäuse selbst ist röntgensichtbar. Ebenso sollte der Auslassbereich der Pumpe, d.h. die Ausströmöffnungen eines Abströmschlauches, im Bereich der aufsteigenden Aorta liegen. Auch dies kann mit einer röntgensichtbaren Markierung überprüft werden. Eine eventuell vorhandene Pigtail-Katheterspitze sollte an der Spitze des linken Herzventrikels anstoßen.

Um die Pumpe aus dem Herzventrikel zu entfernen, wird diese mittels am Wellenkatheter aufgebrachter Zugkraft in die Einführschleuse zurückgezogen und komprimiert aus dem arteriellen Gefäßsystem entfernt. Sie kann auch, wenn die erste Schleuse bereits gekürzt ist, zunächst in den Wellenkatheter ein Stück weit zurückgezogen werden, um die Pumpe zu komprimieren. Hierzu kann der Wellenkatheter einen Einzugtrichter aufweisen, in den die Pumpe durch Zug an der Antriebswelle eingezogen werden kann. Anschließend werden die erste Schleuse und weitere verbliebene Komponenten aus dem Gefäßsystem entfernt.

Besonderen Vorteil bringt bei der Erfindung die Verwendung einer langen Schleuse bei der Implantation und Explantation der Pumpe. Die lange Schleuse dient nicht nur, wie im Stand der Technik üblich, zum Einführen der Pumpe in ein körpereigenes Lumen, sondern zur Führung der Pumpe durch das Schleusenlumen in die Nähe des Wirkungsorts. Hierbei ist es vorteilhaft, wenn im medizinischen Bereich die Schleuse eine Länge zwischen 40 und 120 cm besitzt. Die Länge wird durch den späteren Wirkungsort der Pumpe und die Physis des Patienten bestimmt.

Wird die Pumpe zusammen mit der langen Schleuse aus einem körpereigenen Lumen herausgezogen, so wird die Blutung der femoralen Arterie mit einem Druckverband gestillt. Alternativ kann die Pumpe aus dem Schleusenlumen der langen Schleuse herausgezogen werden. Dann kann durch das Lumen der Schleuse ein weiterer Führungsdraht platziert werden, über den dann nach Entfernung der Schleuse eine Vorrichtung zum Schließen der Punktur geführt werden kann. Hierdurch ist eine verbesserte Blutungsstillung erreichbar.

Die Figuren 10 bis 13 zeigen speziell eine erfindungsgemäße Ausführung der ersten Schleuse mit einer bzw. mehreren Klemmvorrichtungen zur Fixierung eines schlauchförmigen Abschnittes 41 in einem Schleusengehäuse 43.

Fig. 10 zeigt dazu in einem Längsschnitt ein Schleusengehäuse 43, das im Wesentlichen die Form einer zylindrischen Hülse aufweist, die zumindest an dem distalen, dem Patientenkörper zugewandten Ende 44 durch eine Druckschraube 45 abgeschlossen ist. Das Schleusengehäuse 43 weist einen durchgehenden Aufnahmekanal 46 für einen schlauchförmigen Abschnitt 41 der ersten Schleuse auf. In der Darstellung der Fig. 10 ist der schlauchförmige Abschnitt 41 vom Patientenkörper kommend bis in den Spülraum 47 des Aufnahmekanals 46 durchgehend, anschließend in proximaler Richtung gestrichelt dargestellt. Dies deutet an, dass der schlauchförmige Abschnitt 41 gegenüber dem Schleusengehäuse 43 innerhalb des Aufnahmekanals 46 axial verschiebbar ist oder, anders ausgedrückt, dass das Schleusengehäuse 43 auf dem schlauchförmigen Abschnitt 41 verschiebbar ist.

Zum Einführen eines Funktionselementes, beispielsweise einer Pumpe, in die erste Schleuse wird üblicherweise der schlauchförmige Abschnitt 41 so weit in distaler Richtung aus dem Schleusengehäuse 43 herausgezogen oder bei der Herstellung der ersten Schleuse so positioniert, dass er etwa in Höhe des ersten Anschlagstückes 48 endet. Bis zu diesem Punkt kann dann eine zweite Schleuse mit einer eingezogenen Pumpe, wie oben beschrieben, vorgeschoben werden, um dann die Pumpe von der zweiten Schleuse in die erste Schleuse durchzubewegen.

Die erste Klemmvorrichtung weist als Elemente die erste Druckschraube 45, einen ersten Klemmring 50 aus einem Elastomermaterial sowie das erste Anschlagstück 48 auf.

Die Druckschraube ist mittels eines Außengewindes im Bereich der Überlappung mit dem distalen Ende 44 des Schleusengehäuses 43 mit diesem verschraubt. Ein manuelles Drehen an der Druckschraube 45 bewirkt damit eine Bewegung der Druckschraube in axialer Richtung, die zu einer axialen Kompression oder Ausdehnung des Klemmrings 50 führt. Bei einer axialen Kompression hat der Klemmring 50 die Tendenz, zur Erhaltung seines Volumens radial nach innen und außen auszuweichen, und klemmt damit den schlauchförmigen Abschnitt 41, da er auf seiner proximalen Seite einen Widerstand durch das erste Anschlagstück 48 erfährt.

Damit wird der schlauchförmige Abschnitt 41 axial gegenüber dem Schleusengehäuse 43 fixiert. Diese Fixierung kann einfach durch Lösen der Druckschraube 45 aufgelöst werden, so dass der schlauchförmige Abschnitt 41 dann leicht axial in dem Schleusengehäuse 43 verschoben werden kann. Der Klemmring kann dazu im entspannten Zustand einen Innendurchmesser aufweisen, der gleich wie oder größer als der Durchmesser der ersten Schleuse ist.

Wird also zunächst der schlauchförmige Abschnitt 41 möglichst weit in den Patientenkörper hineingeschoben, um ein Einführen der Pumpe in dem Schutz der Schleuse bis zum Einsatzort, beispielsweise in einem Herzventrikel, zu ermöglichen, so wird nach dem Ausbringen der Pumpe der schlauchförmige Abschnitt 41 herausgezogen, und die Schleuse insgesamt steht relativ weit aus dem Patientenkörper vor. Danach kann die Klemmvorrichtung 48, 45, 50 gelöst und das Schleusengehäuse 43 auf dem schlauchförmigen Abschnitt 41 näher zum Patientenkörper herangeschoben werden. Dabei durchsetzt der schlauchförmige Abschnitt 41 das Schleusengehäuse 43 dann vollständig und ragt gegebenenfalls in proximaler Richtung aus diesem heraus. Darauf kann mit Mitteln, die weiter unten ausführlicher beschrieben werden, der schlauchförmige Abschnitt 41 teilweise abgetrennt werden, um die Überlänge zu entfernen.

Innerhalb des Schleusengehäuses 43 ist zur besseren Abdichtung ein sogenanntes kombiniertes Hämostaseventil, bestehend aus einem Domventil 51 und einer Ventilplatte 52 vorgesehen. Die Ventilplatte schließt das Schleusengehäuse 43, wenn an dieser Stelle weder der schlauchförmige Abschnitt 41 noch ein Wellenkatheter den Aufnahmekanal 46 durchsetzt, während das Domventil 51 für den dichten Abschluss um einen strangförmigen Körper herum, beispielsweise den schlauchförmigen Abschnitt oder einen Katheter, optimiert ist.

Am proximalen Ende 53 des Schleusengehäuses 43 ist eine weitere Druckschraube 54 vorgesehen, die im Prinzip ebenso funktioniert wie die erste Druckschraube 45 und die über ein Druckstück 55 die Kompression eines zweiten Klemmrings 56 gegenüber einem zweiten mechanischen Anschlag 57 bewirkt. Als Besonderheit ist hier zu erwähnen, dass der zweite Klemmring 56 an seinem distalen Ende konisch zuläuft, was eine Verformung radial nach innen beim Ausüben eines axialen Drucks durch die Druckschraube 54 begünstigt. Entsprechend gegensinnig ist der zweite Anschlag 57 konisch ausgebildet. Es kann jedoch an dieser Stelle ebenso ein nicht konischer, sondern im Querschnitt rechteckiger oder runder Klemmring 56 eingesetzt werden.

In der Fig. 10 ist eine Spülvorrichtung 58 schematisch angedeutet, die das Spülen des Spülraums 47 mit einer Flüssigkeit ermöglicht, die das Eindringen von Keimen durch die erste Schleuse in den Patientenkörper verhindert. Diese Spülung ist besonders dann effektiv, wenn der schlauchförmige Abschnitt 41 in dem Spülraum 47 oder auf dessen distaler Seite endet, so dass sowohl die Außenseite als auch die Innenseite des schlauchförmigen Abschnitts 41 von der Spülflüssigkeit erreicht werden.

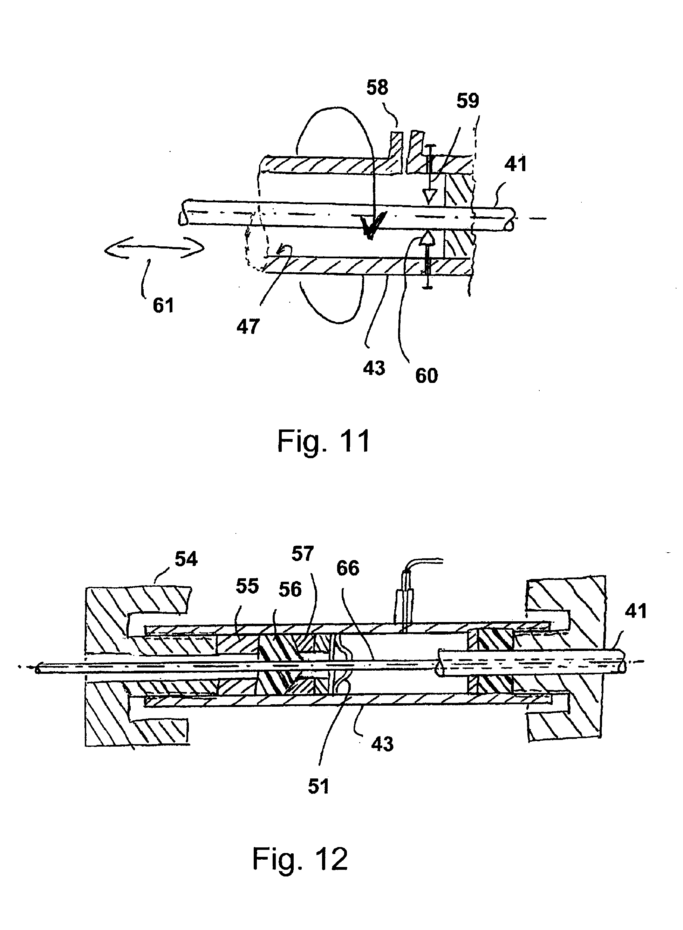

Fig. 11 zeigt exemplarisch die Anordnung und Funktionsweise einer erfindungsgemäßen Schneidvorrichtung.

Wenn keine vorgeschnittenen oder in anderer Weise, beispielsweise durch eine vorgegebene Molekülstruktur oder stellenweise Schwächung der Wandstärke des schlauchförmigen Abschnitts 21, vorgegebenen Sollbruchstellen vorgesehen sind, können diese passend bei Verwendung der ersten Schleuse durch eine Schneidvorrichtung eingebracht werden. In der Fig. 11 ist im Bereich des Spülraumes 47 des Schleusengehäuses 43 eine Schneidvorrichtung mit Klingen 59, 60 vorgesehen, die beispielsweise bei einer Drehung des Schleusengehäuses gegenüber dem schlauchförmigen Abschnitt diesen in Umfangsrichtung schneiden. Es können auch Schnitte in Axialrichtung eingebracht werden.

Zu diesem Zweck können die Klingen 59, 60 auch so angeordnet sein, dass sie bei einer Bewegung des schlauchförmigen Abschnitts 41 in axialer Richtung, wie durch den Pfeil 61 angedeutet, in Längsrichtung schneiden. Es können auch sowohl Klingen für ein Schneiden in Umfangsrichtung als auch eine Klinge für ein Schneiden in Längsrichtung vorgesehen sein.

In der Fig. 11 ist außerdem dargestellt, dass die Klingen 59, 60 in Richtung radial auf den schlauchförmigen Abschnitt 41 zu durch eine Betätigung von der Außenseite des Schleusengehäuses 43 bewegt werden können. Dort können eine in Radialrichtung verlaufende Führung für einen oder mehrere Klingenhalter, eine entsprechende Dichtung und eine Federung vorgesehen sein, so dass das Eindringen von Keimen durch diese Verschiebungsvorrichtung für die Klingen verhindert wird und die Klingen im nicht betätigten Zustand radial einen Abstand zu dem schlauchförmigen Abschnitt 41 einnehmen. Nach der Verwendung der ersten Schleuse kann dann von Hand ein Druck auf die Klingen ausgeübt und der nicht benötigte Teil des schlauchförmigen Abschnitts 41 abgeschnitten werden. Ein hier nicht dargestellter Anschlag verhindert, dass die Schnitttiefe ein kritisches Maß übersteigt und dadurch ein eventuell innerhalb der Schleuse befindlicher Katheter beschädigt wird.

Die dargestellten Klingen können auch eine Schneidvorrichtung für eine zweite Schleuse bilden.

Die Fig. 12 zeigt eine vorteilhafte Verwendung der zweiten Klemmvorrichtung auf der proximalen Seite des Schleusengehäuses 43, nachdem der schlauchförmige Abschnitt 41 gekürzt ist und ein Wellenkatheter 61 aus dem proximalen Ende des schlauchförmigen Abschnitts 41 heraus und weiter zu einer nicht dargestellten Kopplungsvorrichtung für eine antreibbare Welle einer Pumpe aus dem Schleusengehäuse 43 führt. Der Wellenkatheter ist in der oben angesprochenen Domdichtung 51 gedichtet, und die Klemmvorrichtung mit den Elementen der zweiten Druckschraube 54 und dem zweiten Klemmring 56, der durch das Druckstück 55 gegenüber einem zweiten Anschlag 57 axial komprimiert ist, weicht so weit radial nach innen aus, dass er den Wellenkatheter 61, der einen wesentlich geringeren Außendurchmesser aufweist als der schlauchförmige Abschnitt 41 oder eine zweite Schleuse, klemmt und insbesondere auch zusätzlich dichtet. Auf diese Weise können in dem Schleusengehäuse 43 sowohl der schlauchförmige Abschnitt 41 als auch der aus diesem herausragende Wellenkatheter 61 fixiert werden.

Die zweite Klemmvorrichtung ist ebenso dazu geeignet, beim Einführen einer zweiten Schleuse in das Schleusengehäuse 43 die zweite Schleuse mit dem zweiten Klemmring 56 so zu fixieren, dass sie gegenüber dem Schleusengehäuse 43 und insbesondere auch gegenüber dem schlauchförmigen Abschnitt 41 zum Durchschieben des Wellenkatheters 61 ausreichend fixiert ist.

Der erste und zweite Klemmring 50, 56 können aus einem Elastomer, beispielsweise einem Gummi oder einem Silikonelastomer, bestehen und somit vollelastisch, aber volumeninkompressibel verformbar sein. Es ist an dieser Stelle jedoch auch die Verwendung eines elastischen Schaumstoffs denkbar, der teilweise volumenkompressibel ist.

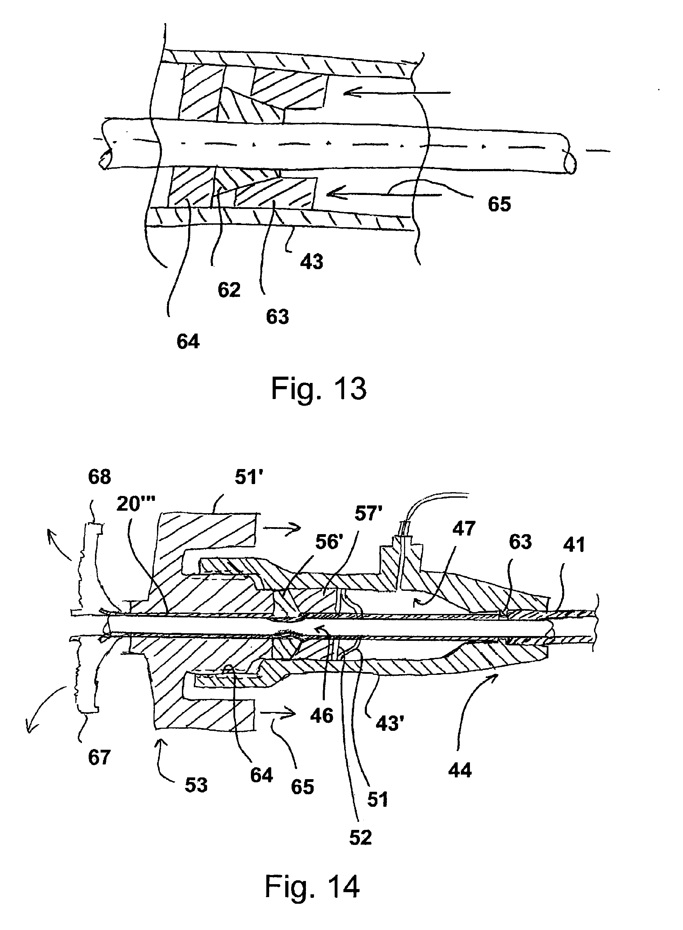

In der Fig. 13 ist schematisch eine andere Art eines Klemmrings 62 gezeigt, der beispielsweise aus einem Kunststoff oder einem Metall bestehen kann und insbesondere geschlitzt und damit radial komprimierbar ist. Der geschlitzte Klemmring 62 weist eine konische Außenkontur auf, gegen die die konische Kontur eines Druckstücks 63 drückt, um den Klemmring radial zu komprimieren, sobald auf das Druckstück 63 eine axiale Druckkraft in Richtung des Pfeils 65, beispielsweise durch eine oben dargestellte Druckschraube, ausgeübt wird. Der geschlitzte Klemmring 62 ist axial durch das Anschlagstück 64 fixiert.

Die Fig. 14 zeigt ein Schleusengehäuse 43', das in seinem Inneren einen Aufnahmekanal 46 für eine Schleuse bzw. einen Katheter aufweist. Das Schleusengehäuse 43' weist an seinem distalen Ende 44 einen an diesem befestigten schlauchförmigen Abschnitt 41 auf, der beispielsweise in einer Öffnung des Schleusengehäuses eingeklebt, eingegossen oder dort anderweitig befestigt sein kann. Der schlauchförmige Abschnitt 41 ist in die Öffnung des Schleusengehäuses 43' bis zu einem mechanischen Anschlag 63 eingeschoben.

Vom proximalen Ende 53 des Schleusengehäuses 43' her ist eine zweite Schleuse 20''' in den Aufnahmekanal 46 so weit eingeschoben, dass sie distal an dem mechanischen Anschlag 63 endet. In einer Ausgestaltung kann das System auch so ausgeführt werden, dass die zweite Schleuse 20''' direkt an dem schlauchförmigen Abschnitt 41 endet. In die zweite Schleuse 20''' ist, nicht mehr dargestellt, beispielsweise ein Funktionselement in Form einer Pumpe mit einem Hohlkatheter eingezogen.

Zur Überführung des Katheters mit der Pumpe von der zweiten Schleuse 20''' in den schlauchförmigen Abschnitt 41 der ersten Schleuse 43', 41 sind die beiden Schleusen innerhalb des Aufnahmekanals 46 koaxial zueinander ausgerichtet, und die zweite Schleuse 20''' ist mittels einer Klemmvorrichtung fixiert. Die Klemmvorrichtung weist einen elastischen Klemmring 56' auf, der an seinem distalen Ende konisch zulaufend ausgebildet ist und gegen einen mechanischen Anschlag 57' gedrückt wird. Zu diesem Zweck wird mittels einer Druckschraube 54', die ein Außengewinde 64 aufweist, axialer Druck auf den Klemmring 56' ausgeübt. Die Druckschraube 54' wird zu diesem Zweck in die Öffnung des röhrenförmigen Teils des Schleusengehäuses 43' eingeschraubt, so dass sie sich axial in die Richtung des Pfeils 65 bewegt.

Der Klemmring 56' besteht beispielsweise aus einem Elastomer, dehnt sich bei axialem Druck in radialer Richtung aus und klemmt somit einen strangförmigen Körper, der sich in dem Aufnahmekanal 46 befindet. Die zweite Schleuse 20''' weist eine Wanddicke zwischen 0,3 und 0,7 mm auf und ist aus einem ausreichend stabilen Material gefertigt, so dass bei radialem Druck die zweite Schleuse geklemmt werden kann, ohne dass gleichzeitig der darin verlaufende Katheter mit eingeklemmt wird. Der Katheter lässt sich somit leicht vom proximalen Ende der zweiten Schleuse 20''' aus in den schlauchförmigen Abschnitt 41 verschieben. Die zweite Schleuse 20"' ist durch eine kombinierte Platten- und Domdichtung 51, 52 in einem Spülraum 47 gedichtet.

Nachdem das Funktionselement, beispielsweise die Pumpe, mit dem Katheter aus der zweiten Schleuse 20''' in den schlauchförmigen Abschnitt 41 überführt worden ist, kann die zweite Schleuse anhand der Handgriffe 67, 68 aufgerissen und entfernt werden. Zu diesem Zweck weist die zweite Schleuse eine Vorschwächung oder einen Einschnitt entlang ihrer Axialrichtung oder eine entsprechend vorgegebene Molekülstruktur auf, die ein Längsaufreißen bis zum distalen Ende der zweiten Schleuse und ein entsprechendes Entfernen der zweiten Schleuse ermöglicht. Zum Aufreißen kann es sinnvoll sein, die Klemmvorrichtung 54', 56', 57' zu lösen.

Nach dem Entfernen der zweiten Schleuse kann die Klemmvorrichtung 54', 56', 57' so weit geklemmt werden, dass der vom Durchmesser kleinere Katheter durch die weitere radiale Kompression des Klemmrings 56' in dem Aufnahmeraum 46 geklemmt wird. Damit ist der Katheter und somit auch eine zu implantierende Pumpe am distalen Ende des Katheters in Axialrichtung gegenüber der ersten Schleuse und damit gegenüber dem Patientenkörper fixiert.

Durch hier nicht dargestellte Ausformungen im Schleusengehäuse wird für die verschiedenen Endpositionen der Druckschraube, die den verschiedenen zu klemmenden Durchmessern entsprechen, jeweils ein spürbarer Anschlag realisiert, so dass der Anwender bei Erreichen der jeweiligen Klemmposition einen deutlich steigenden Drehwiderstand beim Betätigen der Schraube spürt.

Nach einer Anfangsphase, in der die Anordnung sich mechanisch setzt und auf die Körpertemperatur des Patienten erwärmt, kann die Klemmvorrichtung gelöst und der Katheter nachjustiert und darauf wieder fixiert werden. Überall, wo in der beschriebenen Konstruktion zwei zylindrische Elemente dichtend ineinandergesteckt werden, kann vorteilhaft eine Konusdichtung mit einem Konuswinkel von wenigen Grad verwendet werden, wie sie im medizinischen Bereich grundsätzlich bekannt ist.

Die beschriebene Ausführungsform einer Schleuse erlaubt es, beispielsweise eine implantierbare Herzpumpe von einer zweiten Schleuse, in der sie nach einer ersten Inspektion vorrätig gehalten werden kann, in eine erste Schleuse, die in einen Patientenkörper führt, ohne Probleme und mit geringem Aufwand sowie großer Zuverlässigkeit zu überführen.

## Patentansprüche

1. Schleuseneinrichtung zum Einführen eines Katheters in einen Patientenkörper mit einer ersten Schleuse (10, 11, 13, 21, 21', 21", 41, 43, 43') mit einem proximalen Ende und einem distalen Ende, wobei beim bestimmungsgemäßen Gebrauch das distale Ende der ersten Schleuse zur Anordnung im Patientenkörper und das proximale Ende der ersten Schleuse zur Anordnung außerhalb des
Patientenkörpers vorgesehen ist und wobei die erste Schleuse einen schlauchförmigen Abschnitt (11, 21, 21', 21", 41) und ein an dessen proximalem Ende angeordnetes Schleusengehäuse (13, 43, 43') mit einem Aufnahmekanal (46) für einen strangförmigen Körper (20', 20", 20''', 32, 66) aufweist,
**dadurch gekennzeichnet,**
**dass** an dem Aufnahmekanal (46) eine Klemmvorrichtung (54, 54', 55, 56, 56', 57, 57') zum Fixieren eines strangförmigen Körpers in dem Aufnahmekanal durch Klemmen vorgesehen ist.

2. Schleuseneinrichtung nach Anspruch 1, **dadurch gekennzeichnet, dass** die Klemmvorrichtung (54, 54', 55, 56, 56', 57, 57', 62, 63, 64) einen elastisch radial deformierbaren Klemmring (56, 56') aufweist.

3. Schleuseneinrichtung nach Anspruch 1 oder 2,
**dadurch gekennzeichnet, dass** der Klemmring (56, 56') aus einem Elastomer besteht.

4. Schleuseneinrichtung nach Anspruch 1 oder 2,
**dadurch gekennzeichnet, dass** der Klemmring (62) aus einem Kunststoff oder Metall besteht und geschlitzt ist.

5. Schleuseneinrichtung nach Anspruch 2, 3 oder 4, **dadurch gekennzeichnet, dass** der Klemmring (56, 56') mittels einer axial in Bezug auf den Aufnahmekanal wirkenden Schraubvorrichtung (54, 54') in Richtung radial nach innen expandierbar ist.

6. Schleuseneinrichtung nach Anspruch 5, **dadurch gekennzeichnet, dass** die axial wirkende Schraubvorrichtung (54, 54') mittels eines Druckstücks (55) axialen Druck auf den deformierbaren Klemmring (56, 56') ausübt.

7. Schleuseneinrichtung nach Anspruch 5, **dadurch gekennzeichnet, dass** der Klemmring (62) mittels der axial wirkenden Schraubvorrichtung und eines axial bewegbaren Keilkörpers (63), insbesondere eines im Querschnitt keilförmigen Rings, radial nach innen komprimiert wird.

8. Schleuseneinrichtung nach Anspruch 6 oder 7, **dadurch gekennzeichnet, dass** in axialer Richtung gesehen auf der dem Druckstück oder dem Keilkörper gegenüberliegenden Seite des Klemmrings eine radiale Erweiterung des Aufnahmekanals (46) angeordnet ist, die eine Spülvorrichtung aufweist.

9. Schleuseneinrichtung nach Anspruch 6 oder 7, **dadurch gekennzeichnet, dass** auf der in axialer Richtung gesehen dem Druckstück oder dem Keilkörper gegenüberliegenden Seite des Klemmrings (56, 56', 62) ein den Aufnahmekanal abdichtendes Ventil (51, 52) angeordnet ist.

10. Kathetereinrichtung mit einer Schleuseneinrichtung gemäß einem der Ansprüche 1 bis 9 und mit einem Katheter (32, 66), der den Aufnahmekanal (46) durchsetzt und in der Klemmvorrichtung fixiert ist.

11. Kathetereinrichtung mit einer Schleuseneinrichtung gemäß einem der Ansprüche 1 bis 9 und mit einem Katheter, der innerhalb einer zweiten, hülsenförmigen Schleuse den Aufnahmekanal durchsetzt, wobei die zweite Schleuse in der Klemmvorrichtung (54, 54', 55, 56, 56', 57, 57') fixiert ist.

12. Verfahren zum Einführen eines Katheters (32, 66) in einen Patientenkörper mittels einer Schleuseneinrichtung gemäß einem der Ansprüche 1 bis 9 sowie einer zweiten Schleuse (20, 20', 20", 20'''), **dadurch gekennzeichnet, dass** zunächst die zweite Schleuse gemeinsam mit dem Katheter in die erste Schleuse (10, 11, 13, 21, 21', 21", 41, 43, 43'), insbesondere bis zu einem mechanischen Anschlag (63), eingeführt wird, dass darauf die zweite Schleuse mittels der Klemmvorrichtung (54, 54', 55, 56, 56', 57, 57') fixiert wird und dass danach der Katheter von der zweiten Schleuse in die erste Schleuse überführt wird.

13. Verfahren nach Anspruch 12, **dadurch gekennzeichnet, dass** nach dem Einführen des Katheters in die erste Schleuse die zweite Schleuse (20, 20', 20", 20'''), insbesondere durch Auf- oder Abreißen, entfernt wird.

14. Verfahren nach Anspruch 13, **dadurch gekennzeichnet, dass** nach dem Entfernen der zweiten Schleuse (20, 20', 20", 20''') der Katheter in der Klemmvorrichtung (54, 54', 55, 56, 56', 57, 57') fixiert wird.
